# EUROPEAN PATENT APPLICATION

(11) **EP 2 698 161 A1**
(43) Date of publication of application: **19.02.2014**
(21) Application number: 12771869.0
(22) Date of filing: 02.04.2012
(51) Int. Cl.: A61K 36/00, A23L 1/30, A23L 1/302, A23L 2/52, A61K 31/51, A61P 3/10, A61P 43/00

(54) **COMPOSITION, GLUCOSE METABOLISM-IMPROVING AGENT, AND METHOD FOR IMPROVING GLUCOSE METABOLISM**

(30) Priority: 15.04.2011 JP 2011091461
(71) Applicant: Lion Corporation, Tokyo 130-8644 (JP)
(72) Inventor: KAMBAYASHI, Hiroaki, Tokyo 130-8644 (JP); SUZUKI, Noriyuki, Tokyo 130-8644 (JP); MURAKOSHI, Michiaki, Tokyo 130-8644 (JP)
(74) Representative: Graf von Stosch, Andreas
(86) International application number: PCT/JP2012/058819
(87) International publication number: WO 2012/141018

(57) **Abstract**

A composition, including: (A) *Panax notoginseng*; and at least one of (B) a *Piper longum* extract and (C) vitamin B1, a salt thereof or a derivative thereof.

## Description

### Technical Field

The present invention relates to: a composition suitably used for improving glucose metabolism; a food, a beverage, a pharmaceutical drug, a glucose metabolism-improving composition and a glucose metabolism-improving agent, each of which contains the composition; and a method for improving glucose metabolism.

### Background Art

In recent years, Japanese have gradually been obese because their dietary habits have been high-fat and high-caloric, for example. It has been suggested that impaired glucose tolerance, high blood pressure, and so on are greatly related to not only genetic factors but also environmental factors such as obesity. When the metabolic pathway of glucose in the process using glucose as energy does not work normally due to, for example, obesity and as a result failures to metabolize glucose occur such as inhibition of use of glucose, chronic hyperglycemic condition is caused. Such chronic hyperglycemic condition causes systemic vascular disorders and may further cause complicating diseases such as nerve disorders and retinopathy, which is problematic.

One possible measure to prevent or improve obesity is improving daily habits. However, improving daily habits by themselves are often difficult to continue. Therefore, demand has arisen for simple and effective prevention or improvement of glucose metabolism.

Proposed means of improving glucose metabolism include: compositions containing panaxadiol (PD) and/or panaxatriol (PT) contained in *Panax notoginseng* (see PTLs 1 and 2); a composition containing *Panax notoginseng* or an extract thereof irradiated with microwaves (see PTL 3); and granulated products formed by granulating dry powder of *Panax notoginseng*; dry powder of lingzhi mushroom, and dry powder of agaricus mushroom (see PTL 4).

However, even use of these proposed compositions does not exhibit sufficient glucose metabolism-improving effects. Hence, at present, there is a need to provide: a composition having more excellent glucose metabolism-improving effects, having high safety, and capable of being conveniently used, a food, a beverage, a pharmaceutical drug, a glucose metabolism-improving composition and a glucose metabolism-improving agent, each of which contains this composition; and a method for improving glucose metabolism.

### Citation List

### Patent Literature

PTL 1: Japanese Patent Application Laid-Open (JP-A) No. 2011-026314
PTL 2: JP-A No. 2011-026313
PTL 3: JP-A No. 2002-114696
PTL 4: JP-A No. 2000-143526

### Summary of Invention

### Technical Problem

The present invention aims to solve the above existing problems and achieve the following objects. Specifically, an object of the present invention is to provide: a composition having excellent glucose metabolism-improving effects, having high safety, and capable of being conveniently used, a food, a beverage, a pharmaceutical drug, a glucose metabolism-improving composition and a glucose metabolism-improving agent, each of which contains this composition; and a method for improving glucose metabolism.

### Solution to Problem

The present inventors conducted extensive studies to solve the above problems and have obtained the following findings. Specifically, they have found that a composition containing: (A) *Panax notoginseng*; and at least one of (B) a *Piper longum* extract and (C) vitamin B1, a salt thereof or a derivative thereof, has excellent glucose metabolism-improving effects, has high safety, and is capable of being conveniently used. The present invention has been accomplished on the basis of this finding.

The present invention is based on the above finding obtained by the present inventors, and a means for solving the above problems is a composition containing: (A) Panax notoginseng; and at least one of (B) a *Piper longum* extract and (C) vitamin B1, a salt thereof or a derivative thereof

### Advantageous Effects of Invention

The present invention can provide: a composition having excellent glucose metabolism-improving effects, having high safety, and capable of being conveniently used, a food, a beverage, a pharmaceutical drug, a glucose metabolism-improving composition and a glucose metabolism-improving agent, each of which contains this composition; and a method for improving glucose metabolism. These can solve the above existing problems and achieve the above objects.

### Description of Embodiments

### (Composition)

A composition of the present invention contains at least: (A) *Panax notoginseng*; and at least one of (B) a *Piper longum* extract and (C) vitamin B1, a salt thereof or a derivative thereof; and, if necessary, further contains other ingredients.

The state of the above composition is not particularly limited and may be appropriately selected depending on the intended purpose. Examples thereof include a liquid state, a solid state and a semi-solid state.

### <(A) Panax notoginseng>

The *Panax notoginseng* (denshichi ninjin, also called sanshichi ninjin) (hereinafter may be referred to as "ingredient (A)") is a plant belonging to the genus *Panax* of the family *Araliaceae.*

The *Panax notoginseng* may be used as is after it has been harvested from the natural world. Alternatively, the *Panax notoginseng* may be one obtained after its natural product has been subjected to treatments such as washing, drying, cutting, smashing and milling, which are appropriately combined together, and the thus-treated product has been extracted, purified and/or fermented. A commercially available product may be used as the *Panax notoginseng.*

Examples of the commercially available product include *Panax notoginseng* powder and *Panax notoginseng* aqueous extract powder (these products are of MATSUURA YAKUGYO CO., LTD.).

In particular, from the viewpoint of obtaining the glucose metabolism-improving effects, the *Panax notoginseng* is preferably an extract obtained by extracting its powder with a solvent, more preferably an acid-treated product thereof, particularly preferably an acid-treated product containing at least one of panaxadiol (PD) and panaxatriol (PT) at a high concentration.

The panaxadiol or panaxatriol is an aglycon formed after the sugar moiety has been removed from saponin (glycoside) of the *Panax notoginseng* and then the side chain has been ring-closed. It is formed by subjecting the *Panax notoginseng* to acid treatment.

The acid-treated product containing at least one of the panaxadiol and the panaxatriol at a high concentration can exhibit excellent metabolism-improving effects. The acid-treated product containing a mixture of the panaxadiol and the panaxatriol at a high concentration can exhibit more excellent glucose metabolism-improving effects, which is advantageous.

The total amount of the panaxadiol and the panaxatriol in the ingredient (A) is not particularly limited and may be appropriately selected depending on the intended purpose, but is preferably 0.1% by mass or more, more preferably 0.1% by mass to 50% by mass, still more preferably 1% by mass to 50% by mass, particularly preferably 10% by mass to 30% by mass.

The amount of the panaxadiol or the panaxatriol in the ingredient (A) can be measured through gas chromatography using commercially available panaxadiol or panaxatriol as a standard substance.

### <<Method for producing the acid-treated product>>

The method for producing the acid-treated product is not particularly limited and may be appropriately selected depending on the intended purpose. One preferred method includes: a hydrolyzing step of hydrolyzing the *Panax notoginseng* with an aqueous acid solution; a neutralizing step of neutralizing the liquid obtained after the hydrolysis; a filtrating step of filtrating the neutralized liquid; and a drying step of drying the residue after the filtration. The method described in International Publication No. WO2010/029915 can be employed as the method for producing the acid-treated product of the *Panax notoginseng.*

### -Hydrolyzing step-

The hydrolyzing step is a step of hydrolyzing the *Panax notoginseng* with an aqueous acid solution, and is preferably performed in the presence of a lower alcohol.

The aqueous acid solution is not particularly limited and may be appropriately selected depending on the intended purpose. Examples thereof include aqueous solutions containing inorganic acids such as hydrochloric acid, phosphoric acid, sulfuric acid and nitric acid. These may be used alone or in combination of two or more thereof. Among them, an aqueous solution containing hydrochloric acid is preferred.

The concentration of the acid in the aqueous acid solution is not particularly limited and may be appropriately selected depending on the intended purpose, but is preferably 0.04% by mass to 16% by mass, more preferably 2% by mass to 12% by mass. When the concentration of the acid in the aqueous acid solution is less than 0.04% by mass, the hydrolysis is not sufficiently performed and as a result at least one of panaxadiol and panaxatriol may not be formed. Whereas when it is more than 16% by mass, the hydrolysis may excessively proceed and there may be a disadvantage in terms of cost.

The amount of the aqueous acid solution used is not particularly limited and may be appropriately selected depending on the intended purpose. It is preferably 2 times by volume to 20 times by volume relative to the *Panax notoginseng.* When the amount of the aqueous acid solution used is less than 2 times by volume relative to the *Panax notoginseng,* the *Panax notoginseng* is not sufficiently immersed in the aqueous acid solution and as a result the hydrolysis cannot sufficiently be conducted. Whereas when it is more than 20 times by volume, there may be a disadvantage in terms of cost.

The lower alcohol is not particularly limited and may be appropriately selected depending on the intended purpose. Examples thereof include methanol, ethanol and propanol. These may be used alone or in combination of two or more thereof. Among them, ethanol is preferred in terms of safety.

When the lower alcohol is used as an aqueous solution containing the lower alcohol, the mixing ratio between water and the lower alcohol is not particularly limited and may be appropriately selected depending on the intended purpose. The mixing ratio by volume of water : the lower alcohol is preferably 9 : 1 to 2 : 1, more preferably 3 : 1.

The amount of the lower alcohol used is not particularly limited and may be appropriately selected depending on the intended purpose. It is preferably 1% by volume to 80% by volume, more preferably 10% volume to 50% by volume, particularly preferably 20% by volume to 40% by volume, relative to the total amount of the hydrolyzation liquid. When the amount of the lower alcohol used is less than 1% by volume relative to the total amount of the hydrolyzation liquid, sapogenins may not efficiently be formed. Whereas when it is more than 80% by volume, at least one of panaxadiol and panaxatriol may not efficiently be formed and there may be a disadvantage in terms of cost.

Notably, the "total amount of the hydrolyzation liquid" refers to the total amount of the reaction liquid containing the aqueous acid solution and the lower alcohol.

The temperature at which the hydrolysis is performed is not particularly limited and may be appropriately selected depending on the intended purpose. It is preferably 60°C to 100°C, more preferably 70°C to 90°C.

The time for which the hydrolysis is performed is not particularly limited and may be appropriately selected depending on the intended purpose. It is preferably 0.5 hours to 24 hours, more preferably 2 hours to 8 hours.

### -Neutralizing step-

The neutralizing step is a step of neutralizing the hydrolyzed liquid obtained from the hydrolysis.

The method for the neutralization is not particularly limited and may be a known method. Examples thereof include a method in which an aqueous solution of a base such as sodium hydroxide or potassium hydroxide is appropriately added to the hydrolyzed liquid.

Notably, the pH of the liquid after the neutralization is not particularly limited and may be appropriately selected depending on the intended purpose. The pH thereof is preferably 5 to 8.

### -Filtrating step-

The filtrating step is a step of filtrating the hydrolyzed liquid after the neutralizing step, to thereby be separated into a filtrate and a residue.

The method for the filtration is not particularly limited and may be appropriately selected from known methods. Notably, after the filtration, the obtained product may be repeatedly washed with water until the salts are completely removed. The washing with water is preferred also in terms of being able to reduce the concentration of ethanol.

### -Drying step-

The drying step is a step of drying the residue after the filtration.

The method for the drying is not particularly limited and may be appropriately selected from known methods. Examples thereof include freeze drying, air-circulation drying, reduced-pressure drying, spray drying and heating drying.

The amount of the ingredient (A) in the above composition is not particularly limited and may be appropriately selected depending on the intended purpose, but is preferably 3% by mass or more, more preferably 3% by mass to 70% by mass, particularly preferably 6% by mass to 64% by mass. When the amount of the ingredient (A) is less than 3% by mass, the glucose metabolism-improving effects may not be obtained.

### <(B) Piper longum extract>

The *Piper longum* extract (hereinafter may be referred to as "ingredient (B)") is not particularly limited and may be appropriately selected depending on the intended purpose so long as it is an extract of *Piper longum.*

*Piper longum* is an evergreen vine distributed in the Southeast Asia and belonging to the genus *Piper* of the family *Piperaceae.* Its spike becomes a fleshy, thick, cylindrical shape, and is used as a condiment. The part of the *Piper longum* used for the extraction in the present invention is not particularly limited and may be appropriately selected depending on the intended purpose. Examples thereof include spike, roots, leaf, stem, flower, and parts containing these in combination. Among them, spike is particularly preferred.

The ingredient (B) does not exhibit the glucose metabolism-improving effects alone. However, when the ingredient (B) and the ingredient (A) are used in combination, advantageously, the glucose metabolism-improving effects of the ingredient (A) can be further improved, although its mechanism is not known.

The method for extracting the ingredient (B) is not particularly limited and may be appropriately selected depending on the intended purpose. Examples thereof include a method of extracting it with a solvent. The extract may be subjected to treatments such as purification and fermentation, or may be dried. Also, a commercially available product may be used as the ingredient (B).

The solvent used for the extraction is not particularly limited and may be appropriately selected depending on the intended purpose. Examples thereof include water, ethanol and a mixture thereof.

The extraction temperature at which the above extraction is performed is not particularly limited and may be appropriately selected depending on the intended purpose, but is preferably 25°C to 95°C, more preferably 40°C to 80°C.

The extraction time for which the above extraction is performed is preferably 0.1 hours to 12 hours, more preferably 0.5 hours to 4 hours.

Among them, the ingredient (B) is particularly preferably a hot water extract of *Piper longum.*

Specific examples of the commercially available product of the ingredient (B) include LONG PEPPER EXTRACT POWDER MF (trade name) (composition: 10% by mass of a hot water extract of *Piper longum,* 90% by mass of dextrin, product of MARUZEN PHARMACEUTICALS CO., LTD.).

The amount of the ingredient (B) in the above composition is not particularly limited and may be appropriately selected depending on the intended purpose, but is preferably 0.1% by mass to 20% by mass, more preferably 1% by mass to 10% by mass.

The mass ratio ((A) : (B)) of the ingredient (A) and the ingredient (B) in the above composition is not particularly limited and may be appropriately selected depending on the intended purpose, but is preferably 1 : 100 to 1,000 : 1, more preferably 1 : 10 to 100 : 1, particularly preferably 1 : 2 to 20 : 1.

When the mass ratio ((A) : (B)) falls within the above preferred range, advantageously, more excellent glucose metabolism-improving effects can be obtained.

### <(C) Vitamin B1, a salt thereof, or a derivative thereof>

The vitamin B1, salt thereof or derivative thereof (hereinafter may be referred to as "ingredient (C)") is a compound called thiamine having a molecular formula of C₁₂H₁₇N₄OS, a salt thereof, or a derivative thereof.

The salt of the vitamin B1 is not particularly limited and may be appropriately selected depending on the intended purpose. Examples thereof include thiamine hydrochloride, thiamine nitrate, thiamine dicetylsulfate, thiamine thiocyanate, thiamine naphthalene-1,5-disulfonate, thiamine dilaurylsulfate, thiamine disulfide, bisthiamine nitrate, and salts of thiamine dicetylsulfate esters.

The derivative of the vitamin B1 is not particularly limited and may be appropriately selected depending on the intended purpose. Examples thereof include fursultiamine hydrochloride, dibenzoyl thiamine, octothiamine, bisibuthiamine, bisbentiamine, benfothiamine, dicethiamine hydrochloride and cycotiamine.

These may be used alone or in combination of two or more thereof. Among them, thiamine nitrate and thiamine hydrochloride are preferred.

When the ingredient (C) and the ingredient (A) are used in combination, advantageously, the glucose metabolism-improving effects of the ingredient (A) can be further improved, although its mechanism is not known.

The method for obtaining the ingredient (C) is not particularly limited and may be appropriately selected depending on the intended purpose. Examples thereof include: a method of extracting it from vitamin B1, a salt thereof, or a derivative thereof; and a method of synthesizing it. Alternatively, a commercially available product may be used as the ingredient (C).

Specific examples of the commercially available product of the ingredient (C) include vitamin B1 nitrate (trade name) (product of Wako Pure Chemical Industries, Ltd.), vitamin B1 hydrochloride (trade name) (product of AccuStandard Inc.) and thiamine disulfide (trade name) (product of JUNSEI CHEMICAL CO., LTD.).

The amount of the ingredient (C) in the above composition is not particularly limited and may be appropriately selected depending on the intended purpose, but is preferably 0.05% by mass to 10% by mass, more preferably 0.1% by mass to 5% by mass.

The mass ratio ((A) : (C)) of the ingredient (A) and the ingredient (C) in the above composition is not particularly limited and may be appropriately selected depending on the intended purpose, but is preferably 1 : 100 to 10,000 : 1, more preferably 1 : 10 to 1,000 : 1, particularly preferably 1 : 1 to 300 : 1.

When the mass ratio ((A) : (C)) falls within the above preferred range, advantageously, more excellent glucose metabolism-improving effects can be obtained.

The composition of the present invention is not particularly limited and may be appropriately selected depending on the intended purpose so long as it contains: the ingredient (A); and at least one of the ingredient (B) and the ingredient (C). The composition of the present invention preferably contains at least the ingredient (A) and the ingredient (B). Particularly preferably, the composition of the present invention contains all of the ingredient (A), the ingredient (B) and the ingredient (C), since more excellent glucose metabolism-improving effects can be obtained.

When the above composition contains all of the ingredient (A), the ingredient (B) and the ingredient (C), the mass ratio ((B) : (C)) of the ingredient (B) and the ingredient (C) is not particularly limited and may be appropriately selected depending on the intended purpose, but is preferably 1 : 0.01 to 1 : 100, more preferably 1 : 0.1 to 1 : 10. When the mass ratio ((B) : (C)) falls within the above preferred range, advantageously, more excellent glucose metabolism-improving effects of the ingredient (A) can be obtained.

### <Other ingredients>

The other ingredients in the composition are not particularly limited and may be appropriately selected depending on the intended purpose so long as the effects of the present invention are not impaired. Examples thereof include additives, supplements and water.

The additives or supplements are not particularly limited and may be appropriately selected depending on the intended purpose. Examples thereof include a disinfectant, a preserving agent, a binding agent, a thickener, an adhesive agent, an integrating agent, a colorant, a stabilizer, a pH adjuster, a buffer, a tonicity agent, a solvent, an antioxidant, a UV rays-preventing agent, a preventing agent for precipitation of crystals, a defoaming agent, a property improving agent and an antiseptic agent. These may be used alone or in combination of two or more thereof.

The disinfectant is not particularly limited and may be appropriately selected depending on the intended purpose. Examples thereof include cationic surfactants such as benzalkonium chloride, benzethonium chloride and cetylpyridinium chloride.

The preserving agent is not particularly limited and may be appropriately selected depending on the intended purpose. Examples thereof include p-hydroxybenzoate esters, chlorobutanol and clesol.

The binding agent, thickener and adhesive agent are not particularly limited and may be appropriately selected depending on the intended purpose. Examples thereof include starch, dextrin, maltitol, cellulose, methyl cellulose, ethyl cellulose, carboxymethyl cellulose, hydroxyethyl cellulose, hydroxypropyol cellulose, hydroxypropyolmethyl cellulose, carboxymethyl starch, pullulan, sodium alginate, ammonium alginate, propylene glycol alginic acid esters, guar gum, locust bean gum, gum Arabic, xanthane gum, gelatin, casein, polyvinyl alcohol, polyethylene oxide, polyethylene glycol, ethylene/propylene block polymers, sodium polyacrylates and polyvinylpyrrolidone.

The integrating agent is not particularly limited and may be appropriately selected depending on the intended purpose. Examples of the integrating agent include water, ethanol, propanol, simple syrup, glucose liquid, starch liquid, gelatin liquid, carboxymethyl cellulose, hydroxypropyl cellulose, hydroxypropyl starch, methyl cellulose, ethyl cellulose, shellac, calcium phosphate, calcium stearate and polyvinylpyrrolidone.

The colorant is not particularly limited and may be appropriately selected depending on the intended purpose. Examples thereof include titanium oxide and iron oxide.

The stabilizer is not particularly limited and may be appropriately selected depending on the intended purpose. Examples thereof include tragacanth, gum Arabic, gelatin, sodium pyrosulfite, EDTA, thioglycolic acid and thiolactic acid.

The pH adjuster and the buffer are not particularly limited and may be appropriately selected depending on the intended purpose. Examples thereof include sodium citrate, sodium acetate and sodium phosphate.

The tonicity agent is not particularly limited and may be appropriately selected depending on the intended purpose. Examples thereof include sodium chloride and glucose.

The amount of the other ingredients in the composition is not particularly limited and may be appropriately selected depending on the intended purpose.

### <Production method>

The production method for the above composition is not particularly limited and may be appropriately selected depending on the intended purpose. Examples thereof include a method of mixing the ingredient (A) with at least one of the ingredient (B) and the ingredient (C).

When the ingredient (A) and at least one of the ingredient (B) and the ingredient (C) are mixed together, the order of these ingredients, stirring conditions, and mixing conditions such as temperature and humidity are not particularly limited and may be appropriately selected depending on the intended purpose.

### <Applications>

The composition of the present invention has excellent glucose metabolism-improving effects, has high safety, and is capable of being conveniently used. Thus, the composition of the present invention can suitably be used for the below-described food, beverage, pharmaceutical drug, glucose metabolism-improving composition, glucose metabolism-improving agent, and method for improving glucose metabolism of the present invention.

### (Glucose metabolism-improving composition)

A glucose metabolism-improving composition of the present invention contains at least the composition of the present invention; and, if necessary, further contains other ingredients.

### <Composition>

The amount of the above composition in the glucose metabolism-improving composition is not particularly limited and may be appropriately selected depending on the intended purpose so long as the effects of the present invention are not impaired. The glucose metabolism-improving composition may be the above composition itself.

### <Other ingredients>

The other ingredients in the glucose metabolism-improving composition are not particularly limited and may be appropriately selected depending on the intended purpose so long as the effects of the present invention are not impaired. Examples thereof include those similar to the other ingredients in the above composition.

The amount of the other ingredients in the glucose metabolism-improving composition is not particularly limited and may be appropriately selected depending on the intended purpose.

### <Applications>

The composition of the present invention has excellent glucose metabolism-improving effects, has high safety, and is capable of being conveniently used. Thus, the composition of the present invention can suitably be used for the below-described food, beverage, pharmaceutical drug, glucose metabolism-improving agent, and method for improving glucose metabolism of the present invention. Also, the glucose metabolism-improving composition can suitably be used as a blood glucose level-reducing composition since it can especially reduce the high blood glucose level.

### (Food, beverage, pharmaceutical drug)

A food, beverage or pharmaceutical drug of the present invention contains at least the composition of the present invention; and, if necessary, further contains other ingredients.

In the present invention, "food, beverage or pharmaceutical drug" refers to those which are less harmful to human health and which are given orally or through the gastrointestinal tract in the ordinary social life. They are not limited to foods, beverages and pharmaceutical drugs within the administrative boundaries, but include a wide variety of things such as foods including orally-given common foods, healthy foods and health-promoting foods, beverages including common beverages, healthy beverages and health-promoting beverages, and pharmaceutical drugs including ethical pharmaceuticals, proprietary drugs and quasi drugs.

Notably, in the present invention, "food, beverage or pharmaceutical drug" may be collectively referred to as "food or beverage".

### <Composition>

The amount of the composition in the food, beverage or pharmaceutical drug is not particularly limited and may be appropriately selected depending on the type of the food or beverage so long as the effects of the present invention are not impaired. Also, the food or beverage may be the composition itself.

### <Other ingredients>

The other ingredients in the food, beverage or pharmaceutical drug are not particularly limited and may be appropriately selected depending on, for example, the type of foods or beverages to which the composition is to be incorporated. Examples thereof include supplemental materials or additives commonly used for the production of foods, beverages or pharmaceutical drugs.

Examples of the supplemental materials or additives include glucose, fructose, sucrose, maltose, sorbitol, stevioside, rubusoside, corn syrup, lactose, citric acid, tartaric acid, malic acid, succinic acid, lactic acid, L-ascorbic acid, dl-α-tocopherol, sodium erythorbate, glycerin, propylene glycol, glycerin fatty acid esters, polyglycerin fatty acid esters, sucrose fatty acid esters, sorbitan fatty acid esters, gum Arabic, carrageenan, casein, gelatin, pectin, agar, B vitamins other than the ingredient (C), nicotinic-acid amide, calcium pantothenate, amino acids, calcium salts, dyes, perfumes and preservatives.

The amount of the other ingredients contained in the food, beverage or pharmaceutical drug is not particularly limited and may be appropriately selected depending on the intended purpose.

### <Type of food, beverage or pharmaceutical drug>

The type of the food is not particularly limited and may be appropriately selected depending on the intended purpose. Examples thereof include frozen desserts such as ice cream, ice sherbet and ice shavings; noodles such as buckwheat noodles, wheat noodles, vermicelli, coats of Chinese dumplings, coats of pork dumplings, Chinese noodles and instant noodles; snacks such as candy, candies, gum, chocolate, tabletted snacks, munches, biscuits, jelly, jam, cream, baked confectionery and bread; marine products such as crab, salmon, Japanese littleneck, tuna, sardine, shrimps, prawns, bonito, mackerel, whale, oyster, saury, squid, bloody clam, scallop, abalone, sea chestnut, salmon caviar and *Sulculus diversicolor supertexta*; marine/livestock processed foods such as fish minced and steamed, ham and sausage; dairy products such as yogurt; fats and oils or processed foods thereof such as salad oil, *Tempura* oil, margarine, mayonnaise, shortening, whip cream and dressing; seasonings such as sauce and basting; retort pouch foods such as curry, stew, *Oyako-don* (a bowl of rice topped with boiled chicken and eggs), rice porridge, *Zosui* (rice soup), *Chuka-don* (a bowl of rice with a chop-suey-like mixture on it), *Katsu-don* (a rice bowl with pork cutlets), *Ten-don (a tempura* rice bowl), *Una-don* (an eel rice bowl), *hayashi rice* (hashed beef with rice), *Oden* (a dish containing several ingredients such as boiled eggs and radish), mapo doufu, *Gyu-don* (a beef rice bowl), meat sauce, egg soup, rice omelet, Chinese dumplings, pork dumplings, hamburger steak and meat balls; and healthy foods in various forms and dietary supplements.

The type of the beverage is not particularly limited and may be appropriately selected depending on the intended purpose. Examples thereof include beverages such as refreshing beverages, carbonated beverages, energy beverages, fruit beverages and lactic beverage; and dairy products such as processed milk and fermented milk.

The type of the pharmaceutical drug is not particularly limited and may be appropriately selected depending on the intended purpose. Examples thereof include nervous system drugs such as cold reliefs, antipyretic analgesics, cough medicines, sleep improving drugs, sleep-averting drugs, anti-vertiginous drug, pediatric sedatives, drugs for the oral cavity and throat, and mouthwashes; gastrointestinal drugs such as gastrointestinal drug, intestinal remedies, constipating agents, gastrointestinal analgesics and antispasmodics, clysmas, suppositories and anthelmintics; hematological agents such as cardiac stimulants and drugs for anemia; drugs for urinary organs; woman drugs; anti-allergic drugs; revitalizing supplements; various Chinese medicines; drugs for public health; and therapeutic drugs for diabetes.

### (Glucose metabolism-improving agent)

A glucose metabolism-improving agent of the present invention contains at least the composition of the present invention; and, if necessary, further contains other ingredients.

### <Composition>

The amount of the above composition contained in the glucose metabolism-improving agent is not particularly limited and may be appropriately selected depending on the intended purpose so long as the effects of the present invention are not impaired. Also, the glucose metabolism-improving agent may be the above composition itself.

The glucose metabolism-improving agent may be used alone or in combination with another drug containing other active ingredients. The glucose metabolism-improving agent also may be incorporated before use into another drug containing other active ingredients.

### <Other ingredients>

The other ingredients in the glucose metabolism-improving agent are not particularly limited and may be appropriately selected depending on, for example, the dosage form thereof so long as they are pharmacologically acceptable. Examples thereof include those similar to the other ingredients in the above composition.

The amount of the other ingredients contained in the glucose metabolism-improving agent is not particularly limited and may be appropriately selected depending on the intended purpose.

### <Dosage form>

The dosage form of the glucose metabolism-improving agent is not particularly limited and may be appropriately selected depending on the intended administration method. Examples thereof include an oral solid preparation, an oral semi-solid preparation and an oral liquid preparation, with an oral solid preparation being preferred.

### -Oral solid preparation-

The oral solid preparation is not particularly limited and may be appropriately selected depending on the intended purpose. Examples thereof include tablets, chewable tablets, foaming tablets, orally-disintegrating tablets, troches, drops, hard capsules, soft capsules, granules, powder, pills, dry syrups and infusions, with tablets being preferred.

### -Oral semi-solid preparation-

The oral semi-solid preparation is not particularly limited and may be appropriately selected depending on the intended purpose. Examples thereof include electuaries, chewing gums, whip and jelly.

### -Oral liquid preparation-

The oral liquid preparation is not particularly limited and may be appropriately selected depending on the intended purpose. Examples thereof include syrups, drinks, suspensions and spirits.

The production method of the glucose metabolism-improving agent is not particularly limited and may be appropriately selected from known methods depending on, for example, the dosage form thereof.

### <Administration>

The administration method, administration dose, administration period and administration target of the glucose metabolism-improving agent are not particularly limited and may be appropriately selected depending on the intended purpose.

Examples of the administration method include an oral administration method, a parenteral administration method, a local administration method and an enteral administration method.

The administration dose may be appropriately selected considering various factors of an administration target, such as the age, body weight, constitution, symptom and the presence or absence of administration of a drug containing other active ingredients. The total administration dose thereof per day is preferably 0.1 mg to 1,000 mg, more preferably 10 mg to 500 mg, in terms of the amount of the above composition serving as an active ingredient. The glucose metabolism-improving agent may be administered once a day or several times a day in a divided manner.

The animal species serving as the administration target is not particularly limited and may be appropriately selected depending on the intended purpose. Examples thereof include human, monkey, pig, bovine, sheep, goat, dog, cat, mouse, rat and bird, with human being suitably used.

### <Applications>

The glucose metabolism-improving agent of the present invention has excellent glucose metabolism-improving effects, has high safety, and is capable of being conveniently used. For example, the glucose metabolism-improving agent of the present invention can suitably be used for treatment or prevention of abnormal glucose metabolism and for the below-described method for improving glucose metabolism of the present invention.

Also, the above glucose metabolism-improving agent can suitably be used as a blood glucose level-reducing agent since it can especially reduce the high blood glucose level.

### (Method for improving glucose metabolism)

A method for improving glucose metabolism of the present invention includes simultaneously giving (A) *Panax notoginseng*; and at least one of (B) a *Piper longum* extract and (C) vitamin B1, a salt thereof or a derivative thereof.

The ingredient (A), the ingredient (B) and the ingredient (C) used may be the same as those described for the above composition of the present invention, and their preferred examples are also the same as in the above composition of the present invention.

The manner in which the ingredient (A) and at least one of the ingredient (B) and the ingredient (C) are given is not particularly limited and may be appropriately selected depending on the intended purpose so long as they are given simultaneously. The ingredient (A) and at least one of the ingredient (B) and the ingredient (C) are preferably given as, for example, the above composition, glucose metabolism-improving composition, food, beverage, pharmaceutical drug, or glucose metabolism-improving agent of the present invention. These may be used alone or in combination of two or more thereof.

The method for giving the ingredients in the above method for improving glucose metabolism is not particularly limited and may be appropriately selected depending on the intended purpose, but may be in a manner in which the ingredients are given orally or parenterally. Preferably, the ingredients are given orally from in terms of convenience.

In the method for improving glucose metabolism, the amounts of the ingredient (A) and at least one of the ingredient (B) and the ingredient (C) to be given are not particularly limited and may be appropriately selected depending on the intended purpose. They are preferably given in such amounts that the mass ratio ((A) : (B)) of the ingredient (A) and the ingredient (B) is 1 : 100 to 1,000 : 1, more preferably 1 : 10 to 100 : 1, particularly preferably 1 : 2 to 20 : 1.

Also, they are preferably given in such amounts that the mass ratio ((A) : (C)) of the ingredient (A) and the ingredient (C) is 1 : 100 to 10,000 : 1, more preferably 1 : 10 to 1,000 : 1, particularly preferably 1 : 1 to 300 : 1.

Also, they are preferably given in such amounts that the mass ratio ((B) : (C)) of the ingredient (B) and the ingredient (C) is 1 : 0.01 to 1 : 100, more preferably 1 : 0.1 to 1 : 10.

When the mass ratio expressed by at least one of the (A) : (B), the (A) : (C) and the (B) : (C) falls within the above preferred range, advantageously, more excellent glucose metabolism-improving effects can be obtained.

Also, in the method for improving glucose metabolism, the amounts of the ingredient (A) and at least one of the ingredient (B) and the ingredient (C) to be given are not particularly limited and may be appropriately selected depending on the intended purpose. The total amount of the ingredients to be given per day is preferably 0.1 mg to 1,000 mg, more preferably 10 mg to 500. These ingredients may be given once a day or several times a day in a divided manner.

In the method for improving glucose metabolism, the period for which the ingredient (A) and at least one of the ingredient (B) and the ingredient (C) are to be given is not particularly limited and may be appropriately selected depending on the intended purpose.

Since the ingredient (A), the ingredient (B) and the ingredient (C) all have high safety, even giving them for a long period of time is not harmful to health, which is advantageous. Also, there is no problem caused by continuing to give them even after glucose metabolism has been improved.

The animal species serving as the target of the method for improving glucose metabolism is not particularly limited and may be appropriately selected depending on the intended purpose. Examples thereof include human, monkey, pig, bovine, sheep, goat, dog, cat, mouse, rat and bird, with human being suitably used.

### <Applications>

The method for improving glucose metabolism of the present invention exhibits excellent glucose metabolism-improving effects, has high safety, and is capable of being conveniently used. For example, the method for improving glucose metabolism of the present invention can suitably be used for treatment or prevention of abnormal glucose metabolism and be also used as a method for reducing the blood glucose level.

### Examples

The present invention will next be described in detail by way of Examples. The present invention should not be construed as being limited to these Examples. Notably, the amounts of the ingredients shown in tables are values converted to net amounts thereof.

### (Examples 1 to 3 and Comparative Examples 1 to 4)

Mixed feeds of Examples 1 to 3 and Comparative Examples 1 to 4 were prepared by mixing a commercially available feed (trade name: D12450B, product of Research Diet Co.) with *Panax notoginseng* powder (product of MATSUURA YAKUGYO CO., LTD.) and at least one of *Piper longum* extract (trade name, LONG PEPPER EXTRACT POWDER MF, composition: 10% by mass of a hot water extract of *Piper longum,* 90% by mass of dextrin, product of MARUZEN PHARMACEUTICALS CO., LTD.) and vitamin B1 nitrate (product of Wako Pure Chemical Industries, Ltd.) in respective amounts shown in the following Table 1. The above commercially available feed only was used in Comparative Example 4.

Notably, the amounts of panaxadiol and panaxatriol in the *Panax notoginseng* powder were found to be the detection limit or lower when measured using the *Panax notoginseng* powder as a measurement sample by the below-described method. That is, the amounts of panaxadiol and panaxatriol in each of the mixed feeds of Examples 1 to 3 and Comparative Examples 1 to 3 were found to be the detection limit or lower.

Hyperglycemia model mice (TSOD mouse (obtained from Institute for Animal Reproduction), 14 weeks old, 5 mice/group) were given ad libitum each of the mixed feeds of Examples 1 to 3 and Comparative Examples 1 to 4 to breed them for 5 days.

The blood glucose levels at 10 a.m. were measured by the below-described method, before giving the mixed feed to the hyperglycemia model mice (before the start of breeding with the mixed feed) and after giving the mixed feed to the hyperglycemia model mice for 5 days (after the breeding with the mixed feed). Averages of the measured blood glucose levels are shown in the following Table 1. A significance test was performed using the Dunnett's multiple test.

Also, the difference between the blood glucose levels before the start of breeding with the mixed feed and after the breeding with the mixed feed (Δ blood glucose level) was calculated using the following calculation formula.

Δ Blood glucose level (mg/dL) = Blood glucose level before the start of breeding with the mixed feed (mg/dL) - Blood glucose level after the breeding with the mixed feed (mg/dL)

### <Measurement of the blood glucose level>

The blood glucose level was measured with a simplified blood glucose level measuring system (trade name: CYCLIC GB SENSOR, product of Sanko Junyaku Co., Ltd.).

### <Analysis of panaxadiol and panaxatriol>

About 0.1 g of a measurement sample was accurately weighed, and about 8 mL of ethanol (purity: 99.5% by mass) was added to the sample. The mixture was suspended for 15 min using an ultrasonic bath. The suspension was centrifuged at about 700 x g for 10 min, and ethanol (purity: 99.5% by mass) was added to the supernatant so as to have a volume of exactly 10 mL. The thus-prepared liquid was measured through gas chromatography under the following conditions. Notably, in the following conditions, the retention time of panaxadiol was about 18 min, and the retention time of panaxatriol was about 29 min.

### [Analysis conditions]

| | |
|---|---|
| Gas Chromatograph: | GC353B, product of GL Sciences Inc. |
| Detector: | Flame Ionization Detector (FID) |
| Injection method: | Split injection method (split ratio: 1 : 50) |
| Column: | DB-17MS (length: 30 m, inner diameter: 0.25 mm, film thickness: 0.25 µm, product of Agilent Technologies, Ltd.) |
| Column temp.: | Initial temp.: 310°C |
| | Initial temp. retaining time: 20 min |
| | Temperature increasing rate: 10°C/min |
| | Final temp.: 320°C |
| | Final temp. retaining time: 14 min |
| Carrier gas: | Helium |
| Flow rate: | 1.5 mL/min |
| Injection inlet temp.: | 320°C |
| Detector temp.: | 320°C |
| Injection amount: | 1 µL |

**Table 1**

| | Amounts (% by mass) | | | Blood glucose levels (mg/dL) | | Δ Blood glucose level (mg/dL) |
|---|---|---|---|---|---|---|
| | (A) *Panax notoginseng* powder | *(B) Piper longum* extract | (C) Vitamin B1 nitrate | Before the start of breeding with the mixed feed | After the breeding with the mixed feed | |
| Ex. 1 | 1 | 0.083 | 0.1 | 375.6 | 144.4 | 231.2 |
| Ex. 2 | 1 | 0.083 | - | 373.8 | 157.0 | 216.8 |
| Ex. 3 | 1 | - | 0.1 | 376.4 | 201.8 | 174.6 |
| Comp. Ex. 1 | 1 | - | - | 353.8 | 287.4 | 66.4 |
| Comp. Ex. 2 | - | - | 0.1 | 389.2 | 345.3 | 43.9 |
| Comp. Ex. 3 | - | 0.83 | - | 391.7 | 354.2 | 37.5 |
| Comp. Ex. 4 | - | - | - | 399.4 | 451.8 | -52.4 |

From the results of Table 1, it is found that after the breeding with the mixed feed for 5 days, Examples 1 to 3 where the *Panax notoginseng* (ingredient (A)) was given in combination with at least one of the *Piper longum* extract (ingredient (B)) and the vitamin B1 nitrate (ingredient (C)) exhibit significant blood-glucose-level reducing effects at a significance level of lower than 5% as compared with the *Panax notoginsengalone* (Comparative Example 1), the vitamin B1 nitrate alone (Comparative Example 2), the *Panax notoginseng* powder alone (Comparative Example 3) and the commercially available feed alone (Comparative Example 4), confirming improvement in glucose metabolism.

Also, as compared with Example 2 where the *Piper longum* extract (ingredient (B)) only was added to the *Panax notoginseng* (ingredient (A)) and Example 3 where the vitamin B1 nitrate (ingredient (C)) only was added to the *Panax notoginseng* (ingredient (A)), Example 1 where both the *Piper longum* extract (ingredient (B)) and the vitamin B1 nitrate (ingredient (C)) were added to the *Panax notoginseng* (ingredient (A)) exhibits higher blood-glucose-level reducing effects and confirms more excellent glucose metabolism-improving effects.

### (Production Example 1: Production of acid-treated Panax notoginseng product)

5.9% by mass hydrochloric acid (6,666 mL) and 99.9% by mass aqueous ethanol solution (3,334 mL) were mixed with *Panax notoginseng* powder (1,000 g, product of MATSUURA YAKUGYO CO., LTD.). The resultant mixture was hydrolyzed with heating for 6 hours at 80°C to prepare an aglycon-containing extract. Then, the pH of the aglycon-containing extract was adjusted to 6.7 with 6.6M aqueous sodium hydroxide solution to reduce the ethanol concentration, followed by suction filtration. The residue was dried with heating under reduced pressure, to thereby obtain an acid-treated *Panax notoginseng* product containing aglycons.

The acid-treated *Panaxnotoginseng* product was used as a measurement sample and analyzed in the same manner as in Example 1. As a result, the amount of panaxadiol in the acid-treated *Panax notoginseng* product was 5.0% by mass, and the amount of panaxatriol in the acid-treated *Panax notoginseng* product was 7.5% by mass.

### (Examples 4 to 8 and Comparative Examples 5 to 8)

Mixed feeds of Examples 4 to 7 and Comparative Examples 6 to 8 were prepared by mixing a commercially available high fat diet (trade name: Quick Fat, product of CLEA Japan, Inc.) with the acid-treated *Panax notoginseng* product produced in Production Example 1 and at least one of *Piper longum* extract (trade name, LONG PEPPER EXTRACT POWDER MF, composition: 10% by mass of a hot water extract of *Piper longum,* 90% by mass of dextrin, product of MARUZEN PHARMACEUTICALS CO., LTD.) and vitamin B1 nitrate (product of Wako Pure Chemical Industries, Ltd.) in respective amounts shown in the following Table 2. The mixed feed of Example 8 was prepared by using *Panax notoginseng* powder (product of MATSUURAYAKUGYO CO., LTD.) instead of the acid-treated *Panax notoginseng* product of Example 4. The above commercially available high fat diet only was used in Comparative Example 5.

Hyperglycemia model mice (KKAy mice (obtained from CLEA Japan, Inc.), 20 weeks old, 5 mice/group) were given ad libitum each of the mixed feeds of Examples 4 to 8 and Comparative Examples 5 to 8 to breed them for 5 days.

The blood glucose levels at 10 a.m. were measured in the same manner as in Example 1, before giving the mixed feed to the hyperglycemia model mice (before the start of breeding with the mixed feed) and after giving the mixed feed to the hyperglycemia model mice for 5 days (after the breeding with the mixed feed). Averages of the measured blood glucose levels are shown in the following Table 2. A significance test was performed using the Dunnett's multiple test. Also, the Δ blood glucose levels calculated in the same manner as in Example 1 are also given in the following Table 2.

**Table 2**

| | Amounts (% by mass) | | | | Blood glucose levels (mg/dL) | | Δ Blood glucose level (mg/dL) |
|---|---|---|---|---|---|---|---|
| | (A) | | (B) | (C) | Before the start of breeding with the mixed feed | After the breeding with the mixed feed | |
| | Acid-treated *Panax notoginseng* product | *Panax notoginseng* powder | *Piper longum* extract | Vitamin B1 nitrate | | | |
| Ex. 4 | 1 | - | 0.083 | 0.1 | 582 | 246 | 336 |
| Ex. 5 | 1 | - | 0.083 | - | 555 | 276 | 279 |
| Ex. 6 | 1 | - | - | 0.1 | 565 | 328 | 237 |
| Ex. 7 | 7 | - | 0.83 | 0.1 | 573 | 251 | 322 |
| Ex. 8 | - | 1 | 0.83 | 0.1 | 553 | 387 | 166 |
| Comp. Ex. 5 | - | - | - | - | 546 | 590 | -44 |
| Comp. Ex. 6 | 1 | - | - | - | 550 | 406 | 144 |
| Comp. Ex. 7 | - | - | 0.083 | - | 549 | 486 | 63 |
| Comp. Ex. 8 | - | - | - | 0.1 | 551 | 501 | 50 |

From the results of Table 2, it is found that after the breeding with the mixed feed for 5 days, Examples 4 to 7 where the acid-treated *Panax notoginseng* product (ingredient (A)) was given in combination with at least one of the *Piper longum* extract (ingredient (B)) and the vitamin B1 nitrate (ingredient (C)) and Example 8 containing the *Panax notoginseng* powder instead of the acid-treated *Panax notoginseng* product exhibit significant blood-glucose-level reducing effects at a significance level of lower than 5% as compared with Comparative Example 5 (control) using none of the ingredients (A), (B) and (C).

Also, as compared with Comparative Example 6 where the acid-treated *Panax notoginseng* product only was mixed, Examples 4 to 7 exhibit significant blood-glucose-level reducing effects at a significance level of lower than 5%.

In addition, as compared with Example 5 where the *Piper longum* extract (ingredient (B)) only was added to the acid-treated *Panax notoginseng* product (ingredient (A)) and Example 6 where the vitamin B1 nitrate (ingredient (C)) only was added to the acid-treated *Panax notoginseng* product (ingredient (A)), Example 4 where both the *Piper longum* extract (ingredient (B)) and the vitamin B1 nitrate (ingredient (C)) were added to the acid-treated *Panax notoginseng* product (ingredient (A)) exhibits higher blood-glucose-level reducing effects and confirms more excellent glucose metabolism-improving effects.

The composition of the present invention is preferably used as a drinkable preparation or a solid preparation. Formulation Examples (2 examples) will next be given.

### (Formulation Example 1: Drinkable preparation)

The following ingredients of a drinkable preparation were mixed with and dissolved in purified water to obtain 100 mL of a drinkable preparation (pH = 4.5).

### [Ingredients of drinkable preparation]

Acid-treated *Panax notoginseng* product of Production Example 1 (ingredient (A)): 1,000 mg
LONG PEPPER EXTRACT POWDER MF (composition: 10% by mass of a hot water extract of *Piper longum* (ingredient (B)), 90% by mass of dextrin, product of MARUZEN PHARMACEUTICALS CO., LTD.): 1,000 mg
Vitamin B1 nitrate (product of Wako Pure Chemical Industries, Ltd.) (ingredient (C)): 10 mg
Ascorbic acid: 500 mg
Maltitol: 15,000 mg
Vitamin B6: 10 mg
Inositol: 50 mg
Anhydrous caffeine: 20 mg
Malic acid: 150 mg
Citric acid: 700 mg
Sodium citrate: appropriate amount (pH adjuster)
Glycerin: 60 mg
Sodium benzoate: 70 mg
Perfume: appropriate amount
(Formulation Example 2: Tablet)

Gelatin (130 g), glycerin (70 g), water (100 g) and ethyl p-hydroxybenzoate (0.5 g) were stirred under heating to obtain a homogeneous gelatin dispersion liquid (L1).

Separately, enzymatically decomposed lecithin (100 g), dipotassium glycyrrhizate (0.4 g), vitamin B1 nitrate (product of Wako Pure Chemical Industries, Ltd.) (ingredient (C)) (1 g), LONG PEPPER EXTRACT POWDER MF (composition: 10% by mass of a hot water extract of *Piper longum* (ingredient (B)), 90% by mass of dextrin, product of MARUZEN PHARMACEUTICALS CO., LTD.) (10 g) and the acid-treated *Panax notoginseng* product of Production Example 1 (ingredient (A)) (20 g) were dispersed in wheat germ oil (200 g) using HOMOMIXER to prepare wheat germ oil liquid (L2) in the form of homogeneous solution.

The gelatin dispersion liquid (L1) was extruded into a 12 mm-diameter aluminum mold for capsule, to obtain a gelatin capsule.

Next, the wheat germ oil liquid (L2) was extruded with a nozzle to be injected and charged into the gelatin capsule, followed by cooling and drying, to thereby obtain a solid preparation having a gel outer layer of gelatin charged with the wheat germ oil liquid (L2) in the form of liquid (tablet oral drug : gel composition).

The composition per one piece of the above solid preparation is given below.
[Solid preparation/1 piece]
-Outer layer-
Gelatin: 130 mg
Glycerin: 70 mg
Ethyl p-hydroxybenzoate: 0.5 mg
-Inner layer-
Enzymatically decomposed lecithin: 50 mg
Dipotassium glycyrrhizate: 0.4 mg
Wheat germ oil: 100 mg
Vitamin B1 nitrate (ingredient (C)): 1.0 mg
LONG PEPPER EXTRACT POWDER MF (ingredient (B)): 10 mg
Acid-treated *Panax notoginseng* product of Production Example 1 (ingredient (A)): 20 mg

Aspects of the present invention are as follows.
<1> A composition, including:
   (A) *Panax notoginseng*; and
      at least one of (B) a *Piper longum* extract and (C) vitamin B1, a salt thereof or a derivative thereof.
<2> The composition according to <1>, wherein the (A) *Panax notoginseng is* an acid-treated product of the *Panax notoginseng.*
<3> The composition according to <1> or <2>, wherein the (A) *Panax notoginseng* contains at least one of panaxadiol and panaxatriol, and a total amount of the panaxadiol and the panaxatriol is 0.1% by mass to 50% by mass.
<4> A food, a beverage or a pharmaceutical drug, including:
   the composition according to any one of <1> to <3>.
<5> A glucose metabolism-improving composition, including:
   the composition according to any one of <1> to <3>.
<6> A glucose metabolism-improving agent, including:
   the composition according to any one of <1> to <3>.
<7> A method for improving glucose metabolism, the method including:
   simultaneously giving (A) *Panax notoginseng*; and at least one of (B) a *Piper longum* extract and (C) vitamin B1, a salt thereof or a derivative thereof.

### Industrial Applicability

The composition of the present invention, the food, beverage, pharmaceutical drug, glucose metabolism-improving composition, and glucose metabolism-improving agent, each of which contains the composition, and the method for improving glucose metabolism have excellent glucose metabolism-improving effects, have high safety, and are capable of being conveniently used, and thus can suitably be used for treatment or prevention of abnormal glucose metabolism.

## Claims

1. A composition, comprising:
(A) *Panax notoginseng*; and
at least one of (B) a *Piper longum* extract and (C) vitamin B1, a salt thereof or a derivative thereof.

2. The composition according to claim 1, wherein the (A) *Panax notoginseng* is an acid-treated product of the *Panax notoginseng.*

3. The composition according to claim 1 or 2, wherein the (A) *Panax notoginseng* contains at least one of panaxadiol and panaxatriol, and a total amount of the panaxadiol and the panaxatriol is 0.1% by mass to 50% by mass.

4. A food, a beverage or a pharmaceutical drug, comprising:
the composition according to any one of claims 1 to 3.

5. A glucose metabolism-improving composition, comprising:
the composition according to any one of claims 1 to 3.

6. A glucose metabolism-improving agent, comprising:
the composition according to any one of claims 1 to 3.

7. A method for improving glucose metabolism, the method comprising:
simultaneously giving (A) *Panax notoginseng*; and at least one of (B) a *Piper longum* extract and (C) vitamin B1, a salt thereof or a derivative thereof.
